# EUROPEAN PATENT APPLICATION

(11) **EP 4 524 241 A1**
(43) Date of publication of application: **19.03.2025**
(21) Application number: 23889176.6
(22) Date of filing: 09.11.2023
(51) Int. Cl.: C12N 5/0783, C12N 5/0789, A61K 35/17, A61P 35/00

(54) **MEDIUM COMPOSITION FOR INDUCING DIFFERENTIATION FROM PLURIPOTENT STEM CELLS INTO NATURAL KILLER CELLS AND DIFFERENTIATION METHOD USING SAME**

(30) Priority: 10.11.2022 KR 20220149783
(71) Applicant: Sungkwang Medical Foundation, Seoul 06135 (KR)
(72) Inventor: KANG, Eunju, Seoul 04595 (KR); AN, Hee Jung, Seoul 06331 (KR)
(74) Representative: WSL Patentanwälte Partnerschaft mbB
(86) International application number: PCT/KR2023/017981
(87) International publication number: WO 2024/101919

(57) **Abstract**

Provided is a medium composition for inducing differentiation of pluripotent stem cells into natural killer cells. By the medium composition for inducing differentiation of pluripotent stem cells into natural killer cells according to an aspect, the differentiation efficiency into natural killer (NK) cells may be improved, and the expression of NK cell-specific markers that increase ability of NK cells for killing cancer cells may be improved without using an artificial feeder and without any genetic manipulation.

## Description

### Technical Field

The present disclosure relates to a medium composition for inducing differentiation of pluripotent stem cells into natural killer cells.

### Background Art

Currently, general treatments for cancer include surgery, radiation therapy, chemotherapy, and the like, and depending on types and stages of cancer, cancer may be cured by single treatment or a combination of treatments. However, the treatments in either way bring severe side effects and pain to patients. The most ideal cancer treatment would be one capable of selecting and killing cancer cells only while leaving normal cells intact. However, existing treatments cause some damage to normal cells. Cancer immunotherapy is an emerging field of treatment that utilizes the body's own immune system to more specifically eliminate cancer cells with minimal damage to normal cells, and is being actively studied in several subfields (e.g., antibody therapy, immune cell therapy, viral immunotherapy, nanotechnology immunotherapy, etc.). Among these treatments, immune cell therapy is a method of treating cancer by increasing the number of cells, such as natural killer cells, natural killer T cells, T cells, B cells, dendritic cells, and the like, in lymphocytes obtained from the blood of a patient, and then strengthening functions of the cells *in vitro* and returning the cells to the patient's body. Such treatments using immune cells are effective in regulating immune responses, and are evaluated to be excellent in terms of toxicity and safety. Among the immune cells, natural killer (NK) cells are important cells responsible for the innate immunity, and unlike T cells, the NK cells mature in the liver and bone marrow. In particular, the NK cells have the ability to identify and kill abnormal cells, such as virus-infected cells or tumor cells, by themselves. Over the past 10 years, tumor immunotherapies using the immune system of patients have been steadily developing, and 'cell therapy products' associated therewith have also been commercialized. Cell therapy products are defined as pharmaceutical products used for the purposes of treatment, diagnosis, and prevention through a series of actions that change the biological characteristics of cells by proliferating and screening autologous, allogeneic, or xenogeneic cells *in vitro* (see Article 2 of the Ministry of Food and Drug Safety Notification No. 2003-26).

Meanwhile, despite the therapeutic benefits and safety of the NK cells themselves, the NK cells are known as poorly cultured cells, and thus have limitations in mass proliferation. In addition, there are attempts to differentiate pluripotent stem cells into NK cells, but in this case, the efficiency of differentiation into NK cells is low, and the expression of NK cell-specific markers is also low. Thus, methods that can increase the expression through artificial genetic manipulation are being attempted. In addition, when differentiation into NK cells is induced, xenogeneic feeder cells are often used, and thus at the time of preparing a therapeutic agent, the feeder cells may be mixed into a final product, which can cause safety problems.

### Prior Art Documents

### Patent Documents

(Patent Document 1) KR10-2022-0050672
(Patent Document 2) KR10-2019-0060412

### Description of Embodiments

### Technical Problem

One aspect is to provide a medium composition for inducing differentiation of pluripotent stem cells into natural killer cells, the medium composition including: a first medium composition for inducing differentiation of pluripotent stem cells into hematopoietic stem cells; and a second medium composition for inducing differentiation of the hematopoietic stem cells into natural killer cells, wherein the first medium composition includes a GSK inhibitor, bone morphogenetic protein 4 (BMP4), vascular endothelial growth factor (VEGF), basic fibroblast growth factor (bFGF), retinoic acid, a TGF-beta inhibitor, polyvinyl alcohol (PVA), stem cell factor (SCF), or a combination thereof, and the second medium composition includes SCF, interleukin 7 (IL7), interleukin 15 (IL15), FMS-like tyrosine kinase 3 ligand (FLT3L), or a combination thereof.

Another aspect is to provide a kit including the first medium composition and the second medium composition.

Another aspect is to provide a method of inducing differentiation of pluripotent stem cells into natural killer cells, the method including: a first step of inducing differentiation of pluripotent stem cells into hematopoietic stem cells by culturing the pluripotent stem cells in the presence of the first medium composition; and a second step of inducing differentiation of the hematopoietic stem cells into natural killer cells by culturing the hematopoietic stem cells in the presence of the second medium composition.

Another aspect is to provide natural killer cells or a cell population thereof, the natural killer cells being differentiated from pluripotent stem cells by the aforementioned method.

Another aspect is to provide a composition including the natural killer cells or a cell population thereof.

### Technical Solution

One aspect is to provide a medium composition for inducing differentiation of pluripotent stem cells into natural killer cells, the medium composition including: a first medium composition for inducing differentiation of pluripotent stem cells into hematopoietic stem cells; and a second medium composition for inducing differentiation of the hematopoietic stem cells into natural killer cells, wherein the first medium composition includes a GSK inhibitor, bone morphogenetic protein 4 (BMP4), vascular endothelial growth factor (VEGF), basic fibroblast growth factor (bFGF), retinoic acid, a TGF-beta inhibitor, polyvinyl alcohol (PVA), stem cell factor (SCF), or a combination thereof, and the second medium composition includes SCF, interleukin 7 (IL7), interleukin 15 (IL15), FMS-like tyrosine kinase 3 ligand (FLT3L), or a combination thereof.

Another aspect is to provide a kit including the first medium composition and the second medium composition.

Another aspect is to provide a method of inducing differentiation of pluripotent stem cells into natural killer cells, the method including: a first step of inducing differentiation of pluripotent stem cells into hematopoietic stem cells by culturing the pluripotent stem cells in the presence of the first medium composition; and a second step of inducing differentiation of the hematopoietic stem cells into natural killer cells by culturing the hematopoietic stem cells in the presence of the second medium composition.

Another aspect is to provide natural killer cells or a cell population thereof, the natural killer cells being differentiated from pluripotent stem cells by the aforementioned method.

Another aspect is to provide a composition including the natural killer cells or a cell population thereof.

### Advantageous Effects

By the medium composition for inducing differentiation of pluripotent stem cells into natural killer cells according to an aspect, the differentiation efficiency into natural killer cells may be increased, and the expression of NK cell-specific markers may be improved without using an artificial feeder and without any genetic manipulation.

### Description of Drawings

FIG. 1 is a schematic diagram showing a process of differentiating pluripotent stem cells into natural killer cells.
FIG. 2 shows the results of confirming CD markers specific to natural killer cells after inducing differentiation of stem cells into natural killer cells.
FGIS. 3A and 3B show the results of confirming expression levels of surface receptors and cytoplasmic granules of natural killer cells (PSC-NK cells: cells of Example 1, primary NK cells: cells of Comparative Example 3).
FIG. 4 shows the results of evaluating cytotoxicity of natural killer cells in K562 cells (blood cancer cells) and A2780cis cells (ovarian cancer cells) (ES-NK: cells of Example 1, Cryo NK92MI: NK92MI cells).
FIG. 5 shows the results of evaluating cytotoxicity of natural killer cells in SKOV3 cells (ovarian cancer cell line) (ESC-NK: cells of Example 1, CD34+ derived NK: NK cells differentiated from induced pluripotent stem cells, which are generated by using CD34+ cells isolated from UCB, according to methods described in Example 1, Cryo NK92MI: NK92MI cells).

### Best Mode

One aspect provides a medium composition for inducing differentiation of pluripotent stem cells into natural killer cells, the medium composition including: a first medium composition for inducing differentiation of pluripotent stem cells into hematopoietic stem cells; and a second medium composition for inducing differentiation of hematopoietic stem cells into natural killer cells, wherein the first medium composition includes a glycogen synthase kinase 3 (GSK) inhibitor, bone morphogenetic protein 4 (BMP4), vascular endothelial growth factor (VEGF), basic fibroblast growth factor (bFGF), retinoic acid, a transforming growth factor-beta (TGF-beta) inhibitor, polyvinyl alcohol (PVA), stem cell factor (SCF), or a combination thereof, and the second medium composition includes SCF, interleukin 7 (IL7), interleukin 15 (IL15), FMS-like tyrosine kinase 3 ligand (FLT3L), or a combination thereof. The first medium composition and the second medium composition may be used separately for differentiation of pluripotent stem cells into hematopoietic stem cells and differentiation of the hematopoietic stem cells into natural killer cells, respectively.

In an embodiment, the first medium composition may include one or more of the following compositions:
(i) a medium composition including a GSK inhibitor;
(ii) a medium composition including BMP4, VEGF, and bFGF;
(iii) a medium composition including VEGF, bFGF, a TGF-beta inhibitor, and retinoic acid; and
(iv) a medium composition including PVA, SCF, and bFGF.

Here, each of the medium compositions (i) to (iv) may be used separately or simultaneously for differentiation of the hematopoietic stem cells into natural killer cells.

In the present specification, the term "natural killer cells" or "NK cells" may refer to cytotoxic lymphocytes constituting major components of the innate immune system, and are defined as large granular lymphocytes (LGLs) that constitute another cells differentiated from B and T lymphocytes that are produced from common lymphoid progenitors (CLPs). The term "natural killer cells" or "NK cells" may include not only mature natural killer cells, but also precursors of the natural killer cells. The natural killer cells may be activated in response to interferons or macrophage-derived cytokines, and include two types of surface receptors labeled as "activating receptor" and "inhibitory receptor", which control the cytotoxic activity of cells.

The activity of natural killer cells may refer to the occurrence or increase in the function of the natural killer cells, and may refer to the ability to detect and eliminate or kill pathogenic cells, non-specific infectious pathogens, cancer cells, and the like.

In an embodiment, the GSK inhibitor may include the following substances shown in Table 1.

**[Table 1]**

| GSK inhibitor | |
|---|---|
| Metal ion | Beryllium, copper, lithium, mercury, tungsten, zinc, etc. |
| ATP-competitive | - Aminopyrimidines (CHIR99021 CHIR98014, CT98014, CT98023, CT99021, TWS119, etc.) - marine-derived (6-BIO, dibromocantharelline, hymenialdicine, indirubin, meridianin, etc.) |
| | - Arylindolmaleimide (SB-216763, SB-41528, etc.), |
| | - Thiazole (AR-A014418, AZD-1080, etc.) |
| | - Paullones (alsterpaullone, kazpolone, kenpaullone, etc.) |
| | - Aloisine |
| Non-ATP competitive | - Marine-derived (manzamin A, palinurin, tricantin, etc.) - thiazolidinediones (TDZD-8, NP00111, NP031115, tideglusib, etc.) |
| | - Halomethyl ketones (HMK-32, etc.) |
| | - Peptides (L803-mts, L807-mts, etc.) |
| | - Unknown mechanism (COB-187, COB-152, etc.) |

In an embodiment, the TGF-beta inhibitor may include 4-{4-[3-(pyridin-2-yl)-1H-pyrazol-4-yl]-pyridin-2-yl}-N-(tetrahydro-2H-pyran-4-yl)benzamide hydrate, 4-[3-(2-pyridinyl)-1H-pyrazol-4-yl]-quinoline, 2-[3-(6-methyl-2-pyridinyl)-1H-pyrazol-4-yl]-1,5-naphthyridine, 4-(5-benzol[1,3]dioxol-5-yl-4-pyridin-2-yl-1H-imidazol-2-yl)-benzamide hydrate, 4-[4-(1,3-benzodioxol-5-yl)-5-(2-pyridinyl)-1H-imidazol-2-yl]-benzamide hydrate, 4-[4-(3,4-methylenedioxyphenyl)-5-(2-pyridyl)-1H-imidazol-2-yl]-benzamide hydrate, 3-(6-methyl-2-pyridinyl)-N-phenyl-4-(4-quinolinyl)-1H-pyrazole-1-carbothioamide, 2-(3-(6-methylpyridine-2-yl)-1H-pyrazol-4-yl)-1,5-naphthyridine, 4-[3-(2-pyridinyl)-1H-pyrazol-4-yl]-quinoline, 2-[4-(1,3-benzodioxol-5-yl)-2-(1,1-dimethylethyl)-1H-imidazol-5-yl]-6-methyl-pyridine, 2-(5-chloro-2-fluorophenyl)-4-[(4-yridyl)amino]pteridine, 6-[2-tert-butyl-5-(6-methyl-pyridin-2-yl)-1H-imidazol-4-yl]-quinoxaline, 4-{4-[3-(pyridin-2-yl)-1H-pyrazol-4-yl]-pyridine-2-yl}-N-(tetrahydro-2H-pyran-4-yl)benzamide hydrate, 4-[2-fluoro-5-[3-(6-methyl-2-pyridinyl)-1H-pyrazol-4-yl]phenyl]-1H-pyrazole-1-ethanol, 3-[[5-(6-methyl-2-pyridinyl)-4-(6-quinoxalinyl)-1H-imidazol-2-yl]methyl]benzamide, or a combination thereof.

The term "CHIR99021" as used in the present disclosure may refer to a compound having a structural formula of Formula 1:

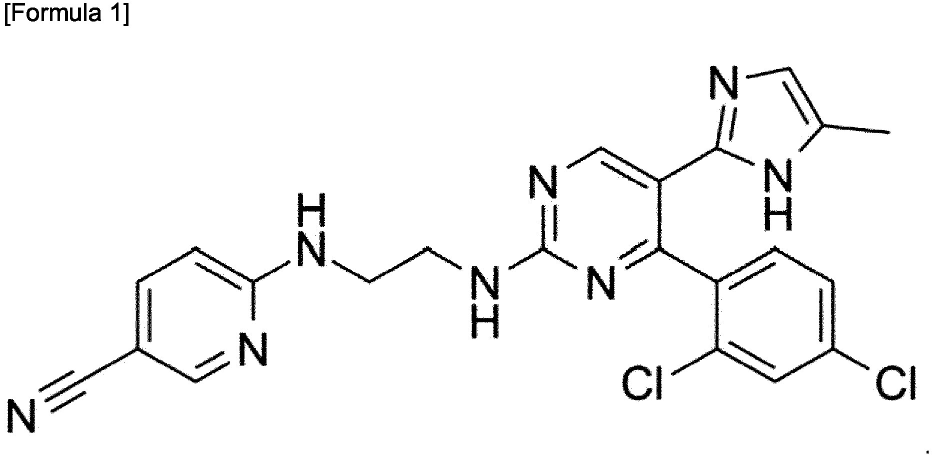

The term "SB-431542" as used in the present disclosure may refer to a compound having a structural formula of Formula 2:

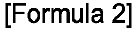

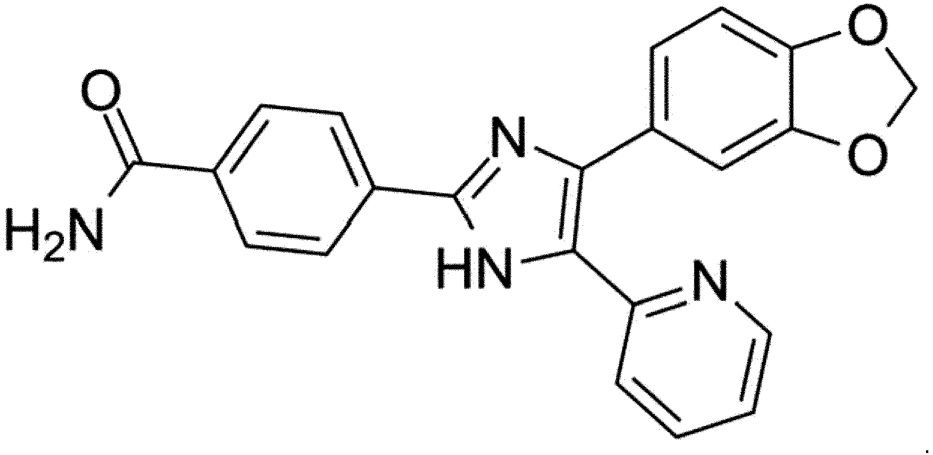

The term "medium" as used in the present disclosure may refer to, although there are differences depending on the purpose of use, a medium for culturing animal cells that basically includes inorganic salts, carbon sources, amino acids, bovine serum albumin (BSA), and cofactors, and the like, and may include all common media well known to those skilled in the art. More preferably, the medium may include inorganic salts, such as NaCl, KCl, NaHCO₃, etc., carbon sources, such as glucose, sodium pyruvate, calcium lactate, etc., amino acids including essential amino acids, such as glutamine, and non-essential amino acids, and cofactors, such as other trace elements, buffers, etc.

The medium may also include antibiotics. For use as the medium, various commercialized media known for culturing animal cells, such as Dulbecco's Modified Eagle's medium (DMEM), endothelial differentiation medium (EDM), minimal essential medium (MEM), Basal Medium Eagle (BME), RPMI1640, F-10, F-12, α-minimal essential medium (α-MEM), Glasgow's minimal essential medium (G-MEM), and Iscove's Modified Dulbecco's Medium, may be used, but embodiments are not limited thereto. In an embodiment, StemPro-34 SFM may be used as the medium.

In an embodiment, a concentration of the GSK inhibitor (e.g., CHIR99021) in the first medium composition may be in a range of 0.1 to 100 µm, 0.1 to 90 µm, 0.1 to 80 µm, 0.1 to 70 µm, 0.1 to 60 µm, 0.1 to 50 µm, 0.1 to 40 µm, 0.1 to 30 µm, 0.1 to 20 µm, 0.1 to 10 µm, 1 to 100 µm, 2 to 100 µm, 3 to 100 µm, 4 to 100 µm, 1 to 90 µm, 2 to 80 µm, 3 to 70 µm, 4 to 60 µm, 4 to 50 µm, 4 to 40 µm, 4 to 30 µm, 4 to 20 µm, 4 to 10 µm, or 1 to 10 µm.

In an embodiment, a concentration of the BMP4 in the first medium composition may be in a range of 1 to 1000 ng/ml, 1 to 900 ng/ml, 1 to 800 ng/ml, 1 to 700 ng/ml, 1 to 600 ng/ml, 1 to 500 ng/ml, 1 to 400 ng/ml, 1 to 300 ng/ml, 1 to 200 ng/ml, 1 to 100 ng/ml, 10 to 1000 ng/ml, 20 to 1000 ng/ml, 30 to 1000 ng/ml, 40 to 1000 ng/ml, 10 to 900 ng/ml, 20 to 800 ng/ml, 30 to 700 ng/ml, 40 to 600 ng/ml, 40 to 500 ng/ml, 40 to 400 ng/ml, 40 to 300 ng/ml, 40 to 200 ng/ml, 40 to 100 ng/ml, or 10 to 100 ng/ml.

In an embodiment, a concentration of the VEGF in the first medium composition may be in a range of 1 to 1000 ng/ml, 1 to 900 ng/ml, 1 to 800 ng/ml, 1 to 700 ng/ml, 1 to 600 ng/ml, 1 to 500 ng/ml, 1 to 400 ng/ml, 1 to 300 ng/ml, 1 to 200 ng/ml, 1 to 100 ng/ml, 10 to 1000 ng/ml, 20 to 1000 ng/ml, 30 to 1000 ng/ml, 40 to 1000 ng/ml, 10 to 900 ng/ml, 20 to 800 ng/ml, 30 to 700 ng/ml, 40 to 600 ng/ml, 40 to 500 ng/ml, 40 to 400 ng/ml, 40 to 300 ng/ml, 40 to 200 ng/ml, 40 to 100 ng/ml, or 10 to 100 ng/ml.

In an embodiment, a concentration of the bFGF in the first medium composition may be in a range of 1 to 1000 ng/ml, 1 to 900 ng/ml, 1 to 800 ng/ml, 1 to 700 ng/ml, 1 to 600 ng/ml, 1 to 500 ng/ml, 1 to 400 ng/ml, 1 to 300 ng/ml, 1 to 200 ng/ml, 10 to 1000 ng/ml, 20 to 1000 ng/ml, 30 to 1000 ng/ml, 40 to 1000 ng/ml, 10 to 900 ng/ml, 20 to 800 ng/ml, 30 to 700 ng/ml, 40 to 600 ng/ml, 40 to 500 ng/ml, 40 to 400 ng/ml, 40 to 300 ng/ml, 40 to 200 ng/ml, or 10 to 200 ng/ml.

In an embodiment, a concentration of the retinoic acid in the first medium composition may be in a range of 0.05 to 50 µm, 0.07 to 50 µm, 0.09 to 50 µm, 0.1 to 50 µm, 0.3 to 50 µm, 0.5 to 50 µm, 0.7 to 50 µm, 0.9 to 50 µm, 0.05 to 40 µm, 0.05 to 30 µm, 0.05 to 20 µm, 0.05 to 10 µm, 0.05 to 5 µm, 0.07 to 40 µm, 0.09 to 30 µm, 0.1 to 20 µm, 0.3 to 20 µm, 0.5 to 10 µm, 0.7 to 5 µm, or 0.1 to 10 µm.

In an embodiment, a concentration of the TGF-beta inhibitor(e.g., SB-431542) in the first medium composition may be in a range of 0.1 to 500 µm, 0.1 to 400 µm, 0.1 to 300 µm, 0.1 to 200 µm, 0.1 to 100 µm, 1 to 500 µm, 5 to 500 µm, 5 to 400 µm, 5 to 300 µm, 5 to 200 µm, 5 to 100 µm, 5 to 50 µm, or 1 to 100 µm.

In an embodiment, a concentration of the PVA in the first medium composition may be in a range of 0.002 to 5 %(w/v), 0.004 to 2 %(w/v), 0.01 to 1 %(w/v), 0.02 to 0.8 %(w/v), 0.02 to 0.5 %(w/v), 0.04 to 0.3 % (w/v), 0.05 to 0.2 %(w/v), or 0.07 to 0.2 %(w/v).

In an embodiment, a concentration of the SCF in the first medium composition may be in a range of 1 to 1000 ng/ml, 1 to 900 ng/ml, 1 to 800 ng/ml, 1 to 700 ng/ml, 1 to 600 ng/ml, 1 to 500 ng/ml, 1 to 400 ng/ml, 1 to 300 ng/ml, 1 to 200 ng/ml, 5 to 500 ng/ml, 10 to 1000 ng/ml, 10 to 500 ng/ml, 10 to 250 ng/ml, 10 to 100 ng/ml, 20 to 100 ng/ml, or 30 to 80 ng/ml.

In an embodiment, in the first medium composition, the concentration of the GSK inhibitor may be in a range of 1 to 10 µM, the concentration of the BMP4 may be in a range of 10 to 100 ng/ml, the concentration of the VEGF may be in a range of 10 to 100 ng/ml, the concentration of the bFGF may be in a range of 10 to 200 ng/ml, the concentration of the retinoic acid may be in a range of 0.1 to 10 µM, the concentration of the TGF-beta inhibitor may be in a range of 1 to 100 µM, the concentration of the PVA may be in a range of 0.01 to 1% (w/v), and the concentration of the SCF may be in a range of 5 to 500 ng/ml.

In an embodiment, a concentration of the SCF in the second medium composition may be in a range of 1 to 100 ng/ml, 1 to 90 ng/ml, 1 to 80 ng/ml, 1 to 70 ng/ml, 1 to 60 ng/ml, 1 to 50 ng/ml, 1 to 40 ng/ml, 1 to 30 ng/ml, 3 to 100 ng/ml, 5 to 100 ng/ml, 7 to 100 ng/ml, 9 to 100 ng/ml, 11 to 100 ng/ml, 13 to 100 ng/ml, 15 to 100 ng/ml, 3 to 90 ng/ml, 5 to 80 ng/ml, 7 to 70 ng/ml, 9 to 60 ng/ml, 11 to 50 ng/ml, 13 to 40 ng/ml, 15 to 30 ng/ml, or 1 to 50 ng/ml.

In an embodiment, a concentration of the IL7 in the second medium composition may be in a range of 1 to 100 ng/ml, 1 to 90 ng/ml, 1 to 80 ng/ml, 1 to 70 ng/ml, 1 to 60 ng/ml, 1 to 50 ng/ml, 1 to 40 ng/ml, 1 to 30 ng/ml, 3 to 100 ng/ml, 5 to 100 ng/ml, 7 to 100 ng/ml, 9 to 100 ng/ml, 11 to 100 ng/ml, 13 to 100 ng/ml, 15 to 100 ng/ml, 3 to 90 ng/ml, 5 to 80 ng/ml, 7 to 70 ng/ml, 9 to 60 ng/ml, 11 to 50 ng/ml, 13 to 40 ng/ml, 15 to 30 ng/ml, or 1 to 50 ng/ml.

In an embodiment, a concentration of the IL15 in the second medium composition may be in a range of 1 to 100 ng/ml, 1 to 90 ng/ml, 1 to 80 ng/ml, 1 to 70 ng/ml, 1 to 60 ng/ml, 1 to 50 ng/ml, 1 to 40 ng/ml, 1 to 30 ng/ml, 1 to 20 ng/ml, 3 to 100 ng/ml, 5 to 100 ng/ml, 7 to 100 ng/ml, 9 to 100 ng/ml, 3 to 90 ng/ml, 5 to 80 ng/ml, 7 to 70 ng/ml, 9 to 60 ng/ml, or 1 to 50 ng/ml.

In an embodiment, a concentration of the FLT3L in the second medium composition may be in a range of 1 to 100 ng/ml, 1 to 90 ng/ml, 1 to 80 ng/ml, 1 to 70 ng/ml, 1 to 60 ng/ml, 1 to 50 ng/ml, 1 to 40 ng/ml, 1 to 30 ng/ml, 1 to 20 ng/ml, 3 to 100 ng/ml, 5 to 100 ng/ml, 7 to 100 ng/ml, 9 to 100 ng/ml, 3 to 90 ng/ml, 5 to 80 ng/ml, 7 to 70 ng/ml, 9 to 60 ng/ml, or 1 to 50 ng/ml.

In an embodiment, in the second medium composition, the concentration of the SCF may be in a range of 1 to 50 ng/ml, the concentration of the IL7 may be in a range of 1 to 50 ng/ml, the concentration of the IL15 may be in a range of 1 to 50 ng/ml, and the concentration of the FLT3L may be in a range of 1 to 50 ng/ml.

In the present specification, the expression "positive" or "+" may refer, with respect to a cell label (marker), that a cell with a label is present in a greater amount or at a higher concentration than other reference cells to be compared. When a cell is with a label inside or on the surface of the cell and the cell can distinguish itself from one or more other cell types by using this label, the cell may become positive to this label. Also, this expression may refer that a cell is with a label in sufficient quantity to produce a signal, e.g., a signal from a cell counting device, at a value greater than a background value. For example, a cell may be labeled to be detectably by an antibody that is specific to CD56, and when a signal from this antibody is detected at a higher level than a signal from a control group (e.g., a background value), the cell may be designated as "positive for CD56" or "CD56+". The expression "negative" or "-" may refer that, even if an antibody that is specific to a certain cell surface label is used, the label is not detected compared to a background value. For example, if a cell is labeled with an antibody that is specific to CD3, but is not detectable, the cell may be designated as "negative for CD3" or "CD3-".

In an embodiment, in a total cell population of the natural killer cells differentiated in the medium composition for inducing differentiation of pluripotent stem cells into natural killer cells of the present disclosure, 10 %, 20 %, 30 %, 40 %, 50 %, 60 %, 70 %, 80 %, 90 %, 95 %, 97 %, or 99 % or more of the cells may be negative for CD3 and positive for CD56.

In an embodiment, in the population of the natural killer cells used herein,

NKG2D may be expressed by 50 %, 60 %, or 64 % or more of CD3⁻CD56⁺ cells, and/or

NKp30 may be expressed by 50 %, 70 %, 80 %, or 89 % or more of CD3⁻CD56⁺ cells, and/or

NKp44 may be expressed by 50 %, 80 %, 90 %, or 94 % or more of CD3⁻CD56⁺ cells, and/or

NKp46 may be expressed by 50 %, 60 %, or 67 % or more of CD3⁻CD56⁺ cells, and/or DNAM-1 may be expressed by 50 %, 80 %, 90 %, or 94 % or more of CD3⁻CD56⁺ cells.

In an embodiment, in the population of the natural killer cells used herein,

NKG2A may be expressed by 5 %, 2 %, or 1 % or less of CD3⁻CD56⁺ cells, and/or

KIR2DL1 may be expressed by 5 %, 2 %, or 1 % or less of CD3⁻CD56⁺ cells, and/or

KIR2DL2/3 may be expressed by 20 %, 15 %, or 10 % or less of CD3⁻CD56⁺ cells, and/or

KIR3DL1 may be expressed by 10 %, 6 %, 5 %, or 3 % or less of CD3⁻CD56⁺ cells.

In an embodiment, in the population of the natural killer cells used herein,
perforin may be expressed by 50 %, 80 %, 90 %, or 98 % or more of CD3⁻CD56⁺ cells, and/or
granzyme B may be expressed by 50 %, 80 %, 90 %, or 98 % or more of CD3⁻CD56⁺ cells.

In an embodiment, the pluripotent stem cells may be embryonic stem cells (ESCs) or induced pluripotent stem cells (iPSCs). Specifically, ESCs or iPSCs may be derived from humans, but embodiments are not limited thereto.

In the present disclosure, the "pluripotent stem cells (PSCs)" may refer to self-renewal stem cells that are pluripotent or totipotent, which can differentiate into cells of all tissues of an individual, and may include ESCs or iPSCs. The "ESCs" may refer to cells cultured *in vitro* by extracting the inner cell mass from an early embryo right before the implantation of a fertilized egg into the mother's uterus, and may be either pluripotent or totipotent capable of differentiating into cells of all tissues of an individual. In a broad sense, the ESCs may also include embryoid bodies derived from the ESCs. The 'iPSCs' may refer to cells that have been induced to have pluripotent differentiation ability through an artificial dedifferentiation process from differentiated cells, and are also called dedifferentiated stem cells. The artificial dedifferentiation process may be performed by introducing: a virus-mediated vector using retrovirus and lentivirus, or a non-viral vector; or a non-virus-mediated dedifferentiation factor using a protein, a cell extract, etc., or may include a dedifferentiation process using a stem cell extract, a compound, etc. The iPSCs may have almost the same characteristics as the ESCs. Specifically, the iPSCs may have similar expression patterns of cells and proteins, have pluripotency *in vitro* and *in vivo,* form teratomas, form a chimera mouse when inserted into mouse blastocysts, and enable germline transmission of genes.

In an embodiment, the medium composition may further include a third medium composition for maturing and expanding the natural killer cells, the third medium composition including IL15, a Rho-associated protein kinase (ROCK) inhibitor, or a combination thereof.

In an embodiment, the ROCK inhibitor may include fasudil, AT-13148, BA-210, β-elemene, belumosudil, chroman 1, DJ4, GSK-576371, GSK429286A, H-1152, hydroxyfasudil, ibuprofen, LX-7101, netarsudil, RKI-1447, ripasudil, TCS-001, thiazovivin, verosudil (AR-12286), Y-27632, Y-30141, Y-33075, Y-39983, or a combination thereof.

In an embodiment, a concentration of the ROCK inhibitor (e.g., fasudil) in the third medium composition may be in a range of 1 to 100 µm, 1 to 90 µm, 1 to 80 µm, 1 to 70 µm, 1 to 60 µm, 1 to 50 µm, 1 to 40 µm, 1 to 30 µm, 1 to 20 µm, 3 to 100 µm, 5 to 100 µm, 7 to 100 µm, 9 to 100 µm, 3 to 90 µm, 5 to 80 µm, 7 to 70 µm, 9 to 60 µm, or 1 to 50 µm.

In an embodiment, a concentration of the IL15 in the third medium composition may be in a range of 1 to 200 µg/ml, 1 to 100 µg/ml, 2 to 200 µg/ml, 5 to 80 µg/ml, 5 to 60 µg/ml, 5 to 40 µg/ml, 5 to 30 µg/ml, 10 to 80 µg/ml, 10 to 60 µg/ml, 10 to 40 µg/ml, or 10 to 30 µg/ml.

In an embodiment, in the third medium composition, the concentration of the IL15 may be in a range of 2 to 200 ng/ml and the concentration of the ROCK inhibitor may be in a range of 1 to 100 µg/ml.

Another aspect provides a method of inducing differentiation of pluripotent stem cells into natural killer cells, the method including: a first step of inducing differentiation of pluripotent stem cells into hematopoietic stem cells by culturing the pluripotent stem cells in the presence of a first medium composition including a GSK inhibitor, BMP4, VEGF, bFGF, retinoic acid, a TGF-beta inhibitor, PVA, SCF, or a combination thereof; and a second step of inducing the hematopoietic stem cells into natural killer cells by culturing the hematopoietic stem cells in the presence of a second medium composition including SCF, IL7, IL15, FLT3L, or a combination thereof.

In an embodiment, the first medium composition may be replaced with a first medium composition having different ingredients, during the first step of inducing differentiation.

In an embodiment, the first medium composition may include one or more of the following compositions:
(i) a medium composition including a GSK inhibitor;
(ii) a medium composition including BMP4, VEGF, and bFGF;
(iii) a medium composition including VEGF, bFGF, a TGF-beta inhibitor, and retinoic acid; and
(iv) a medium composition including PVA, SCF, and bFGF.

In an embodiment, the first step may include the following steps:
(1) culturing cells in a medium composition including a GSK inhibitor:
(2) culturing cells in a medium composition including BMP4, VEGF, and bFGF:
(3) culturing cells in a medium composition including VEGF, bFGF, a TGF-beta inhibitor, and retinoic acid: and
(4) culturing cells in a medium composition including PVA, SCF, and bFGF.

Here, steps (1) to (4) may be performed sequentially, in reverse order, or simultaneously. When these steps are performed simultaneously, cells may be cultured in a medium composition including all of the ingredients of the medium composition of each step.

In an embodiment, the medium composition may include the bFGF at different concentrations for each step.

In an embodiment, the first step may be performed for at least 3 days, 3 to 25 days, 5 to 20 days, or 12 to 16 days.

In an embodiment, step (1) may be performed for 1 to 4 days, step (2) may be performed for 1 to 4 days, step (3) may be performed for 0.5 to 2 days, and/or step (4) may be performed for 2 to 24 days.

In an embodiment, the second medium composition may include SCF, IL7, IL15, and FLT3L.

In an embodiment, the second step may be performed for at least 3 days, 3 to 25 days, 5 to 20 days, or 12 to 16 days. In an embodiment, the method of inducing differentiation may further include a third step of maturing and expanding the natural killer cells by culturing the natural killer cells in the presence of a third medium composition including IL15, a ROCK inhibitor(e.g., fasudil), or a combination thereof.

In an embodiment, the third step may be performed for at least 1 day, 1 to 28 days, 2 to 25 days, 3 to 21 days, 5 to 20 days, or 7 to 14 days.

In an embodiment, the medium composition used herein may include each ingredient at various concentration combinations.

Another aspect provides natural killer cells or a cell population thereof differentiated from pluripotent stem cells by the method of inducing differentiation described herein. The natural killer cells may be as described above.

Another aspect provides natural killer cells or a cell population thereof differentiated from pluripotent stem cells by the method of inducing differentiation described herein.

In an embodiment, a pharmaceutical composition for preventing or treating cancer may be provided, the pharmaceutical composition including, as an active ingredient, natural killer cells or a cell population thereof differentiated by the method of inducing differentiation.

The "cancer" may refer to a tumor, a blood cancer, or a solid cancer, and may include impairing synergistic activity of natural killer cells of an individual or not causing synergistic activity of natural killer cells as target cells under certain conditions. In an embodiment, the cancer may be at least one selected from the group consisting of glioma, gastrointestinal stromal tumor, leukemia, breast cancer, uterine cancer, cervical cancer, stomach cancer, colon cancer, prostate cancer, ovarian cancer, lung cancer, laryngeal cancer, rectal cancer, liver cancer, gallbladder cancer, pancreatic cancer, kidney cancer, skin cancer, bone cancer, muscle cancer, adipose cancer, fibroblast cancer, blood cancer, lymphoma, and multiple myeloma.

In the present specification, the term "prevention" may refer to any action of inhibiting or delaying the occurrence of cancer by administering the composition of the present disclosure.

In the present specification, the term "treatment" may refer to or include alleviation, progression inhibition, or prevention of disease, disorder, or condition or one or more symptoms thereof, and the term "active ingredient" or "pharmaceutically effective amount" may refer to any amount of a composition used in the process of practicing the invention provided herein, the amount being sufficient for alleviation, progression inhibition, or prevention of disease, disorder, or condition or one or more symptoms thereof.

The composition may further include other immune antigen adjuvants that have been already known in the art, and the other immune antigen adjuvants may preferably include one of monophosphoryl lipid (MPL A) and glucopyranosyl lipid adjuvant (GLA-SE, formulated in a stable nano-emulsion of squalene oil-in-water).

A way of administering the pharmaceutical composition is not particularly limited. However, depending on the purpose of use, the pharmaceutical composition may be administered parenterally, such as intravenously, subcutaneously, intraperitoneally, by inhalation, or topically, or orally. A dosage may vary according to weight, age, gender, health status, and diet of a patient, an administration time, an administration method, an excretion rate, severity of a disease, and the like. A daily dosage refers to an amount of a therapeutic agent according to an aspect sufficient to treat an alleviated disease status by administration to a subject in need of treatment. An effective amount of a therapeutic agent may vary according to a particular compound, a disease status and severity thereof, and a subject in need of treatment, and may be generally determined by one of ordinary skill in the art. As a non-limiting example, the dosage of the composition according to an aspect for the human body may vary according to age, weight, gender, and a health status of a patient, a dosage form, and severity of a disease. Based on an adult patient weighing 70 kg, the composition may be, for example, fractionally administered once or several times a day at regular time intervals under conditions of about 1,000 to 10,000 cells/time, 1,000 to 100,000 cells/time, 1,000 to 1,000,000 cells/time, 1,000 to 10,000,000 cells/time, 1,000 to 100,000,000 cells/time, 1,000 to 1,000,000,000 cells/time, or 1,000 to 10,000,000,000 cells/time. The composition may be administered several times at regular time intervals.

The pharmaceutical composition may include a pharmaceutically acceptable carrier and/or additive. For example, the pharmaceutical composition may include sterile water, physiological saline, general buffer (e.g., phosphoric acid, citric acid, other organic acids, etc.), a stabilizer, a salt, an antioxidant (e.g., ascorbic acid, etc.), a surfactant, a suspending agent, an isotonic agent, or a preservative. For topical administration, the pharmaceutical composition may also include organic substances, such as biopolymers, inorganic substances, such as hydroxyapatite, specifically collagen matrices, polylactic acid polymers or copolymers, polyethylene glycol polymers or copolymers, and a combination of chemical derivatives of the foregoing. When the pharmaceutical composition according to an embodiment is formulated in a dosage suitable for injection, immune cells or substances that increase the activity of the immune cells may be dissolved in a pharmaceutically acceptable carrier or may be frozen in a dissolved solution state.

The pharmaceutical composition may include a suspending agent, a solubilizing agent, a stabilizer, an isotonic agent, a preservative, an adsorption inhibitor, a surfactant, a diluent, an excipient, a pH adjuster, an analgesic agent, a buffer, a reducing agent, an antioxidant, and the like, when necessary depending on the administration method or formulation types of the pharmaceutical composition. Pharmaceutically acceptable carriers and agents suitable for the present invention, including those exemplified above, are described in detail in the document [Remington's Pharmaceutical Sciences, 19th ed., 1995]. The pharmaceutical composition may be prepared in a unit dosage form by formulation using a pharmaceutically acceptable carrier and/or an excipient, or may be prepared in a multidose container, according to methods that can be easily carried out by those skilled in the art to which the present disclosure pertains. Here, the formulation may be in the form of a solution, suspension, or emulsion in an oily or aqueous medium, or in the form of a powder, a granule, a tablet, or a capsule.

In an embodiment, a pharmaceutical composition for preventing or treating an infectious disease may be provided, the pharmaceutical composition including, as an active ingredient, natural killer cells or a cell population thereof differentiated by the method of inducing differentiation.

In the present specification, the term "disease" may refer to a pathological condition, particularly cancer, infectious disease, inflammatory disease, metabolic disease, autoimmune disorder, degenerative disease, apoptosis-related disease, and graft rejection.

In the present specification, the term "infectious disease" may collectively refer to infectious diseases caused by organisms such as bacteria, viruses, fungi, and the like. For example, in the case of viral infections such as Human Immunodeficiency Virus (HIV), Epstein-Barr virus (EBV), Human Herpes Virus (HHV), Influenza A virus (IAV), and COV-19, activating receptors of the natural killer cells, such as NKp46, NKp44, or NKp30, may bind to viral glycoproteins to be activating, thereby inducing the death of infected cells. In addition, for example, in the case of viral infections such as cytomegalovirus (CMV) and encephalomyocarditis virus (EMCV), FASL and TRAIL of the natural killer cells may bind to death receptors increased in virus-infected cells to induce the death of infected cells.

Another aspect provides a kit for inducing differentiation of pluripotent stem cells into natural killer cells, the kit including: a first medium composition for inducing differentiation of pluripotent stem cells into hematopoietic stem cells; and a second medium composition for inducing differentiation of the hematopoietic stem cells into natural killer cells, wherein the first medium composition includes a GSK inhibitor, BMP4, VEGF, bFGF, retinoic acid, TGF-beta inhibitor, PVA, SCF, or a combination thereof, and the second medium composition includes SCF, IL7, IL15, FLT3L, or a combination thereof. The first medium composition, the second medium composition, and each ingredient are as described above.

The first medium composition and the second medium composition used herein may be included separately in the kit. In addition, when the first medium composition includes medium compositions (i) to (iv), the medium compositions (i) to (iv) may also be included separately from one another in the kit.

Another aspect provides use of the first medium composition for inducing differentiation of pluripotent stem cells into hematopoietic stem cells, the first medium composition including a GSK inhibitor, BMP4, VEGF, bFGF, a retinoic acid, a TGF-beta inhibitor, PVA, SCF, or a combination thereof.

Another aspect provides use of the second medium composition for inducing differentiation of the hematopoietic stem cells into natural killer cells, the second medium including SCF, IL7, IL15, FLT3L, or a combination thereof.

Another aspect provides use of a third medium composition for maturing and expanding the natural killer cells, the third medium composition including IL15, a ROCK inhibitor, or a combination thereof.

The first medium composition, the second medium composition, the third medium composition, and each ingredient are as described above.

### [Mode for Invention]

Hereinafter, the present disclosure will be described in detail with reference to Examples below. However, these Examples are for illustrative purposes only, and the scope of the present disclosure is not intended to be limited by these Examples.

Terms or words as used in the specification and claims of the present disclosure not to be construed as limited to ordinary or dictionary meaning, but should be interpreted as having meanings and concepts consistent with the technical idea of the disclosure, based on the principle that the inventor can appropriately define the concept of the terms to explain the disclosure of the inventor in the best method.

Throughout the specification of the present disclosure, when a portion "includes" an element, another element may be further included, rather than excluding the existence of the other element, unless otherwise described.

Throughout the specification of the present disclosure, "A and/or B" means A or B, or A and B.

### Example 1: Natural killer cells derived from human pluripotent stem cells

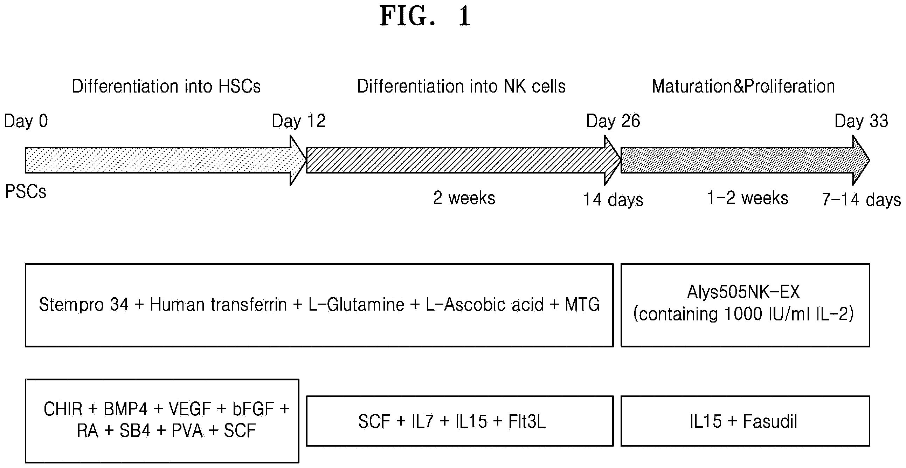

FIG. 1 is a schematic diagram showing a process of differentiating pluripotent stem cells into natural killer cells. Specifically, human pluripotent stem cells were cultured and differentiated into hematopoietic stem cells, and the hematopoietic stem cells were then cultured and differentiated into natural killer cells. Following the differentiation into the natural killer cells, the natural killer cells were matured and expanded.

### 1.1. Differentiation into hematopoietic stem cells (HSCs)

Human pluripotent stem cells (hPSCs) were maintained in a stemMACS-iPS brew medium for 2 days. For a differentiation basal medium, a medium supplemented with stempro34-SFM (+stempro sup) + 200 µg/ml of human transferrin + 2 mM L-glutamine + 0.5 mM L-ascobic acid + 0.45 mM MTG (1-thioglycerol) + 1 % penicillin/streptomycin was used.

The cells were treated with CHIR99021, which is a GSK inhibitor known to induce differentiation into mesenchyma, alone at a concentration of 5 µM for 2 days. Additionally, the cells were treated for another 2 days in the differentiation medium to which 50 ng/ml of BMP4, 50 ng/ml of VEGF, and 100 ng/ml of bFGF were added. Afterwards, the cells were cultured in a culture medium supplemented with 50 ng/ml of VEGF, 50 ng/ml of bFGF, 10 µM of SB-431542, and 1 µM of retinoic acid for another 1 day. From Day 5 to Day 12 of the culture, the cells were cultured in the culture medium to which 0.1 % (w/v, g/ml) PVA, 50 ng/ml of SCF, and 10 ng/ml of bFGF were added, while changing the cell culture medium with a fresh one every day.

### 1.2. Differentiation into natural killer cells

For use as a differentiation basal medium used after the induction into HSCs, the same culture medium as the one used for the induction into HSCs was used. 20 ng/ml of SCF, 20 ng/ml of IL-7, 10 ng/ml of IL-15, and 10 ng/ml of FLT3L were added to the differentiation basal medium, and the HSCs were cultured for 2 weeks, while changing the cell culture medium with a fresh one every two days.

### 1.3. Maturation and expansion of natural killer cells

Following the differentiation into the natural killer cells, only the floating cells were collected and cultured in a cell culture dish for 1 or 2 weeks with a basal culture medium supplemented with Alys505NK-EX (containing 1,000 IU/ml of IL-2) + 10 %(w/v) FBS, wherein 0 µM fasudil, which is as an ROCK inhibitor, and 20 µg/ml of IL-15 were added to the basal culture medium.

The natural killer cells of Example 1 were obtained through the methods described herein.

### Comparative Examples 1 to 4: Natural killer cells isolated from human peripheral blood

According to the methods described in the document [Jung, D., Baek, YS, Lee, IJ et al. Ex vivo expanded allogeneic natural killer cells have potent cytolytic activity against cancer cells through different receptor-ligand interactions. J Exp Clin Cancer Res 40, 333 (published in 2021)], the natural killer cells were isolated.

### Isolation of natural killer cells

Human peripheral blood mononuclear cells (PBMCs) were collected from four healthy donors by leukapheresis. All the healthy donors provided written consent before the study began. CD3-CD56+ primary NK (pNK) cells were obtained by selecting CD3-/CD56+ using anti-CD3 antibodies (Miltenyi Biotec, Bergisch Gladbach, Germany) and anti-CD56 antibodies (Miltenyi Biotec) with Prodigy(Miltenyi Biotec) which is a closed and automatic platform.

The isolated primary NK cells were regarded as cells of Comparative Example 1. Cells obtained by cryopreservation of the isolated primary NK cells were regarded as cells Comparative Example 2.

### Activation, expansion, and freezing of NK cells

The NK cells of Comparative Example 2 were inoculated into a flask coated with NK cell-activating γ-globulin (Green-cross, Yongin, Korea) and anti-NKp46 (R&D Systems, Minneapolis, MN, USA), and then cultured in Alys505NK-EX serum-free medium (CSTI, Sendai, Japan) supplemented with 1,000 IU/mL of recombinant human IL-2 (Novartis, Basel, Switzerland), 50 ng/mL of recombinant human IL-18 (R&D system, Minneapolis, MN, USA), and 5 % heat-inactivated autologous plasma. A fresh culture medium was added every 1 to 3 days according to the cell density (2 × 10⁶ cells/mL). On Day 6, the cells were transferred to a culture bag (NIPRO, Osaka, Japan) and cultured therein for 14 days. The cultured NK cells were then cryopreserved with Cryostor CS5 (BioLife Solutions, Bothell, WA, USA). The cells transferred to the culture bag and cultured therein for 14 days were regarded as cells of Comparative Example 3, and cells obtained by cryopreservation of the cells of Comparative Example 3 were regarded as cells of Comparative Example 4.

### Comparative Example 5: NK-92MI

NK-92MI, a human NK cell line, was purchased from the American Type Culture Collection (ATCC, USA).

### Experimental Example 1: Identification of NK cell-specific markers

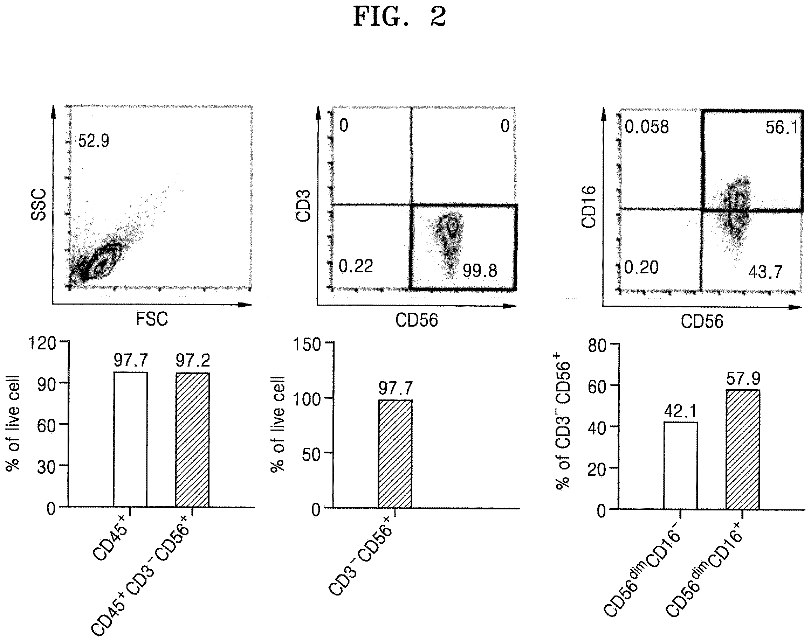

To determine whether the cells obtained in Example 1 differentiated into NK cells, NK cell-specific CD markers were identified by using a fluorescence-activated cell sorting device (FACS, BD FACSCalibur^{™}). Specifically, all cells were harvested at Day 33 of the total NK cell differentiation and analyzed for the CD markers. The results are shown in FIG. 2.

As shown in FIG. 2, it was confirmed that 97 % or more of the cells being analyzed differentiated into CD45+ peripheral leukocytes, and 97 % or more of the cells were identified as CD3-CD56+ cells that are positive to NK cell-specific markers. In addition, it was confirmed that CD16 which induces the antibody-dependent cellular cytotoxicity (ADCC) function of NK cells was expressed by 56 %.

Referring to these results, it was confirmed that the composition according to the present disclosure was able to effectively differentiate pluripotent stem cells into NK cells.

### Experimental Example 2: Confirmation of phenotype of NK cells

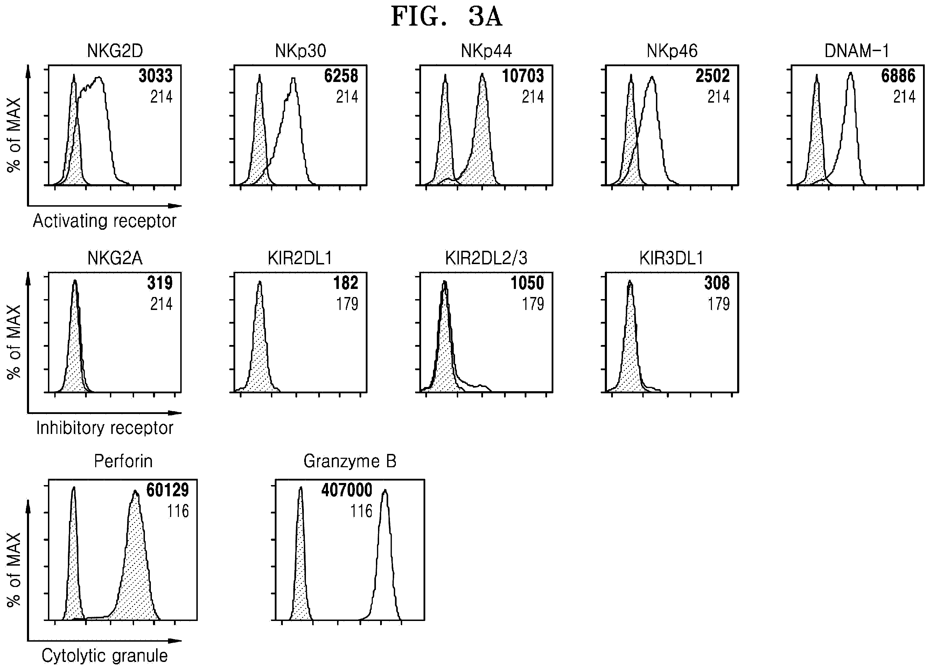

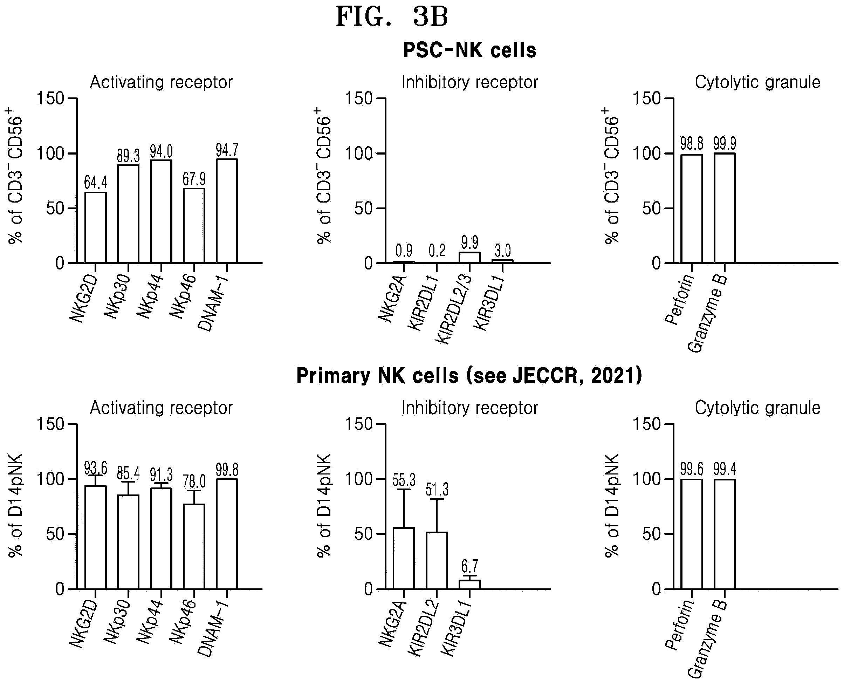

The expression levels of surface receptors and cytoplasmic granules of the cells obtained in Example 1 were confirmed by flow cytometry. To compare with the primary NK cells, the same analysis was performed by using the cells of Comparative Examples 3 and 5. The results are shown in FIGS. 3A and 3B.

As shown in FIGS. 3A and 3B, it was confirmed that the expression of activating receptors of the cells of Example 1 was 64 % for NKG2D, 89 % for NKp30, 94 % for NKp46, and 94 % for DNAM-1 in the CD56+ cells, and that the expression of inhibitory receptors was 1 % or less for NKG2A and KIR2DL1, 9 % for KIR2DL2/3, and 3 % for KIR3DL1 in the CD56+ cells.

Regarding the cytolytic granules, it was confirmed that the expression of perforin and the expression of granzyme B were 98 % or more and 99 % or more, respectively.

The cells of Example 1 showed slightly low expression of the activating receptors (64 % to 94 %) compared to the activated primary NK cells of Comparative Example 3, but showed a relatively high expression rate compared to the NK-92MI (NKp46, 4 %; NKp44, 27 %; DNAM-1, 4 %) of Comparative Example 5. It was confirmed that the inhibitory receptors of the cells of Example 1 were expressed at a much lower level than the primary NK cells. It was confirmed that the cytoplasmic granules of the cells of Example 1 were highly expressed, and thus had characteristics similar with those of the primary NK cells.

Accordingly, it was confirmed that the NK cells of Example 1 had characteristics superior to those of the NK cells of Comparative Example 5, and characteristics similar with or superior to those of the activated NK cells of Comparative Example 3.

### Experimental Example 3: Confirmation of cytotoxicity

### 3-1. K562 cell line and A2780cis cell line

The cytotoxicity was measured for K562 cells (a human chronic myelogenous leukemia cell line) and A2780cis cells (an ovarian cancer cell line) as cisplatin-resistant ovarian cancer cells, wherein these cells have been mainly used to measure the activity of NK cells based on high sensitivity to NK cells. First, target cancer cells (K562 cells or A2780cis cells) were collected and centrifuged at 1500 rpm for 5 minutes, and the supernatant was removed therefrom. Next, the resulting cell pellets were diluted and washed with DPBS, and the washed cell pellets were suspended in a culture medium containing 10 % FBS in a phenol red-free RPMI medium. 1 × 10⁵ cells were prepared for each condition, and left in an incubator to be stained with CFSE (Life technologies) at a concentration of 5 µM under 5 % CO₂ condition for 10 minutes. Afterwards, the cells were washed twice with DPBS and diluted in a culture medium containing 10 % FBS in a phenol red-free RPMI medium. Activated NK cells were prepared at an effector cell to target cell (E:T) (0:1, 1:1, 5:1, and 10:1), and then dispensed and mixed with target cells in a 24-well plate. Then, the activated NK cells and the target cells were allowed for a reaction for 4 hours, and 20 minutes before the end of the reaction, the cells were treated with 7-aminoactinomycin D (7AAD). Even after completion of the reaction, the cells were collected in a 5 ml FACS tube and subjected to analysis of killing ability by using a flow cytometer.

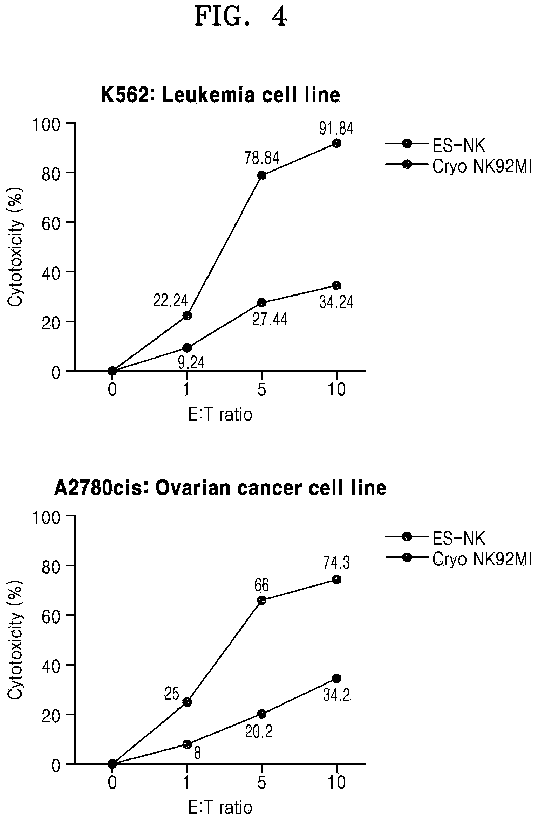

The results are shown in FIG. 4 and Table 2.

As shown in Table 2, the NK cells of Example 1 showed 91 % cytotoxicity at a 10:1 ratio against the K562 cell line that is a blood cancer cell line, and 74% cytotoxicity against the A2780cis cell line that is an anticancer drug-resistant ovarian cancer cell line. The activated primary NK cells of Comparative Examples 3 and 4 exhibited cytotoxicity of 93.22 % and 80.5 % against the K562 cell line, respectively, at a ratio of 10:1, and the primary NK cells of Comparative Example 2 exhibited cytotoxicity of 17.1 % against the K562 cell line at a ratio of 10:1. From these results, it was confirmed that the NK cells of Example 1 had cytotoxicity similar with or superior to cytotoxicity of the primary NK cells of Comparative Examples 2 to 4.

In addition, as shown in FIG. 4, it was confirmed that the cells of Example 1 showed significantly high cytotoxicity compared to the NK92MI cells of Comparative Example 5, indicating that the cells of Example 1 had excellent cytotoxicity.

**[Table 2]**

| Cytotoxicity (%) (E:T=10:1) | Example 1 | Comparative Example 2 | Comparative Example 3 | Comparative Example 4 | Comparative Example 5 |
|---|---|---|---|---|---|
| K562 | 91.84 | 17.1 | 93.22 | 80.5 | 34.24 |
| A2780cis | 74.3 | - | 60.2 | 58.5 | 34.2 |

| | | | | | |
|---|---|---|---|---|---|
| - means 'not-measured'. | | | | | |

Accordingly, it was confirmed that the NK cells of Example 1 had superior characteristics to other NK cells of Comparative Example 5, and had similar or superior characteristics compared to the activated primary NK cells of Comparative Examples 3 and 4.

### 3-2. SKOV3 cell line

The cytotoxicity assay for measuring activity of NK cells described in Experimental Example 3-1 was performed on SKOV3 cells (ovarian cancer cell line). Specifically, the cytotoxicity of NK cells was analyzed by reacting activated NK cells and SKOV3 cells at E:T ratios (0:1, 1:1, 5:1, and 10:1).

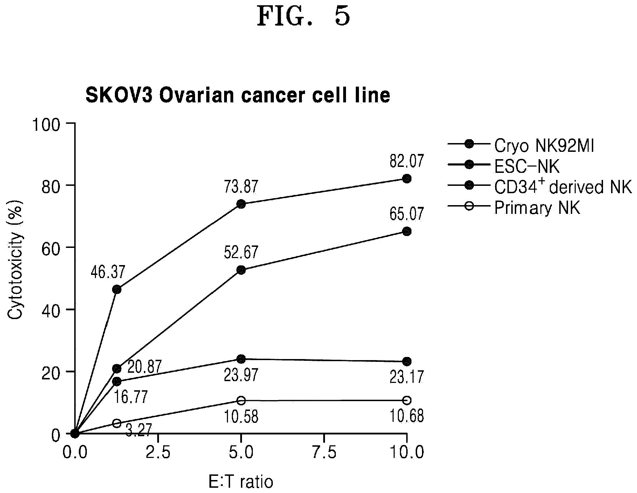

As a result, the primary NK cells or NK92MI cells showed low cytotoxicity against the SKOV3 cell line, whereas the NK cells (ESC-NK) and iPSC-derived NK cells (CD34+ derived NK cells) of Example 1 differentiated according to the method of the present disclosure showed very high cytotoxicity of 65 to 82 % against the SKOV3 cell line at an E:T ratio of 10:1 (FIG. 5). Here, the CD34+ derived NK cells are NK cells differentiated from iPSCs, which are generated by using CD34+ cells isolated from UCB, according to the method described in Example 1. From these results, it is confirmed that the NK cells differentiated from ESCs or iPSCs according to the method of the present disclosure have superior cytotoxicity to other NK cells.

## Claims

1. A kit for inducing differentiation of pluripotent stem cells into natural killer cells, the kit comprising:
a first medium composition for inducing differentiation of pluripotent stem cells into hematopoietic stem cells, comprising a GSK inhibitor, bone morphogenetic protein 4 (BMP4), a vascular endothelial growth factor (VEGF), basic fibroblast growth factor (bFGF), retinoic acid, a TGF-beta inhibitor, polyvinyl alcohol (PVA), stem cell factor (SCF), or a combination thereof; and
a second medium composition for inducing differentiation of the hematopoietic stem cells into natural killer cells, comprising SCF, interleukin 7 (IL7), interleukin 15 (IL15), FMS-like tyrosine kinase 3 ligand (FLT3L), or a combination thereof.

2. The kit of claim 1, wherein, in the first medium composition, a concentration of the GSK inhibitor is 1 to 10 µM, a concentration of the BMP4 is 10 to 100 ng/ml, a concentration of the VEGF is 10 to 100 ng/ml, a concentration of the bFGF is 10 to 200 ng/ml, a concentration of the retinoic acid is 0.1 to 10 µM, a concentration of the TGF-beta inhibitor is 1 to 100 µM, a concentration of the PVA is 0.01 to 1% (w/v), and a concentration of the SCF is 5 to 500 ng/ml.

3. The kit of claim 1, wherein, in the second medium composition, a concentration of the SCF is 1 to 50 ng/ml, a concentration of the IL7 is 1 to 50 ng/ml, a concentration of the IL15 is 1 to 50 ng/ml, and a concentration of the FLT3L is 1 to 50 ng/ml.

4. The kit of claim 1, wherein at least 90 % of cells in a total cell population of the differentiated natural killer cells are negative for CD3 and positive for CD56.

5. The kit of claim 1, wherein the pluripotent stem cells are embryonic stem cells (ESCs) or induced pluripotent stem cells (iPSCs).

6. The kit of any one of claims 1 to 5, wherein the first medium composition and the second medium composition are included separately in the kit.

7. The kit of claim 1, further comprising a third medium composition for maturing and expanding the natural killer cells, comprising IL15, a ROCK inhibitor, or a combination thereof.

8. The kit of claim 7, wherein a concentration of the ROCK inhibitor in the third medium composition is 1 to 50 µM.

9. A method of inducing differentiation of pluripotent stem cells into natural killer cells, the method comprising:
a first step of inducing differentiation of pluripotent stem cells into hematopoietic stem cells by culturing pluripotent stem cells in the presence of a first medium composition comprising a GSK inhibitor, bone morphogenetic protein 4 (BMP4), vascular endothelial growth factor (VEGF), basic fibroblast growth factor (bFGF), retinoic acid, a TGF-beta inhibitor, polyvinyl alcohol (PVA), stem cell factor (SCF), or a combination thereof; and
a second step of inducing differentiation of the hematopoietic stem cells into natural killer cells by culturing the hematopoietic stem cells in the presence of a second medium composition comprising SCF, interleukin 7 (IL7), interleukin 15 (IL15), FMS-like tyrosine kinase 3 ligand (FLT3L), or a combination thereof.

10. The method of claim 9, wherein the first step is performed for 5 to 20 days.

11. The method of claim 9, wherein the second step is performed for 5 to 20 days.

12. The method of claim 9, further comprising a third step of maturing and expanding the natural killer cells by culturing the natural killer cells in the presence of a third medium composition comprising IL15, a Rock inhibitor, or a combination thereof.

13. The method of claim 12, wherein the third step is performed for 5 to 20 days.

14. A natural killer cell or a cell population thereof, the natural killer cell being differentiated from a pluripotent stem cell by the method according to any one of claims 9 to 13.

15. A pharmaceutical composition for preventing or treating cancer, the pharmaceutical composition comprising, as an active ingredient, natural killer cells or a cell population thereof, the natural killer cells being differentiated from pluripotent stem cells by the method according to any one of claims 9 to 13.

16. The pharmaceutical composition of claim 15, wherein the cancer is at least one selected from the group consisting of glioma, gastrointestinal stromal tumor, leukemia, breast cancer, uterine cancer, cervical cancer, stomach cancer, colon cancer, prostate cancer, ovarian cancer, lung cancer, laryngeal cancer, rectal cancer, liver cancer, gallbladder cancer, pancreatic cancer, kidney cancer, skin cancer, bone cancer, muscle cancer, adipose cancer, fibroblast cancer, blood cancer, lymphoma, and multiple myeloma.

17. A pharmaceutical composition for preventing or treating an infectious disease, the pharmaceutical composition comprising, as an active ingredient, natural killer cells or a cell population thereof, the natural killer cells being differentiated from pluripotent stem cells by the method according to any one of claims 9 to 13.

18. A composition for inducing differentiation of pluripotent stem cells into natural killer cells, the composition comprising:
a first medium composition for inducing differentiation of pluripotent stem cells into hematopoietic stem cells, comprising a GSK inhibitor, bone morphogenetic protein 4 (BMP4), vascular endothelial growth factor (VEGF), basic fibroblast growth factor (bFGF), retinoic acid, a TGF-beta inhibitor, polyvinyl alcohol (PVA), stem cell factor (SCF), or a combination thereof; and
a second medium composition for inducing differentiation of the hematopoietic stem cells to natural killer cells, comprising SCF, interleukin 7 (IL7), interleukin 15 (IL15), FMS-like tyrosine kinase 3 ligand (FLT3L), or a combination thereof.
